# FASCICULE DE BREVET EUROPEEN

(11) **EP 0 540 377 B1**
(45) Date de publication et mention de la délivrance du brevet: **10.06.1998**
(21) Numéro de dépôt: 92402602.4
(22) Date de dépôt: 23.09.1992
(51) Int. Cl.: G01N 31/22, G01N 33/48

(54) **Dispositif pour le contrôle des gaz**
Vorrichtung zur Überwachung von Gasen
Device for monitoring gases

(30) Priorité: 24.09.1991 FR 9112272
(43) Date de publication de la demande: 05.05.1993
(73) Titulaire: PONSY, Jacques, 34150 Arboras (FR)
(72) Inventeur: PONSY, Jacques, 34150 Arboras (FR)
(74) Mandataire: Boivin, Claude

(56) Documents cités:
- WO-A-85/04016
- WO-A-91/12527
- FR-A- 2 497 954
- US-A- 3 437 449

## Description

On connaît par le document WO-A-85 04 016 un dispositif pour déterminer le taux d'une substance donnée dans un fluide, par passage d'un volume déterminé de ce fluide dans un appareil de mesure (9) par l'intermédiaire d'un tube à essai (1) qui contient une matière ayant une réaction colorée avec cette substance et est, au stockage, disposé dans un tube de protection (4) qui a sensiblement même longueur que le tube d'essai et est obturé par un opercule imperméable (5).

Au cours des transports, la matière réactive qui est relativement friable, se met en poudre, ce qui nuit au fonctionnement du dispositif.

La présente invention a pour objet un perfectionnement apporté à ces tubes de contrôle dans le but d'éviter cet inconvénient et de simplifier la mise en oeuvre de l'épreuve de contrôle.

Le dispositif selon l'invention est caractérisé en ce que l'opercule (5) est fixé à la fois au tube à essai (1) et au tube de protection (2), de façon à immobiliser ces deux tubes l'un par rapport à l'autre et que la liaison de ce tube d'essai (1) avec l'appareil de mesure (9) s'effectue directement par traversée de l'opercule scellé (5).

Dans un mode de réalisation préféré de l'invention, l'extrémité du tube à essai destinée à se trouver du côté de l'extrémité ouverte du tube de protection est tronconique et présente une bossette extérieure au dessus d'un trou latéral. On peut ainsi sortir le tube d'essai de son tube protecteur, sans contact manuel, à l'aide d'un élément cylindrique dont le diamètre intérieur est sensiblement égal au diamètre extérieur du tube d'essai et dont le bord extérieur est aminci.

On a décrit ci-après, à titre d'exemple non limitatif, un mode de réalisation du dispositif selon l'invention, avec référence au dessin annexé dans lequel :
La Figure 1 est une vue en coupe montrant le tube d'essai enfermé dans son tube de protection;
Les Figures 2 et 3 montrent comment le tube d'essai est extrait du tube de protection;
La Figure 4 montre le tube d'essai en position d'utilisation, dans une application particulière.

Au dessin, on voit un tube d'essai 1 qui contient une masse réactive poreuse 2 destinée à être traversée par un fluide à contrôler et immobilisée axialement par des disques perforés 3; cette masse est par exemple constituée par un gel de silice imbibé d'une solution réactive.

Lors de son stockage, le tube 1 est logé dans un tube de protection en verre 4 qui a pratiquement la même longueur que le tube 1 et est fermé par un opercule imperméable 5 thermo-scellé. Cet opercule assure l'étanchéité et presse sur l'extrémité du tube 1; ce dernier est ainsi immobilisé dans le tube 4 et empêché de ballotter, ce qui risquerait de produire un effritement du gel 2.

L'extrémité 1a du tube 1 est de forme tronconique et présente une bossette extérieure 6 placée au-dessus d'un trou latéral 7.

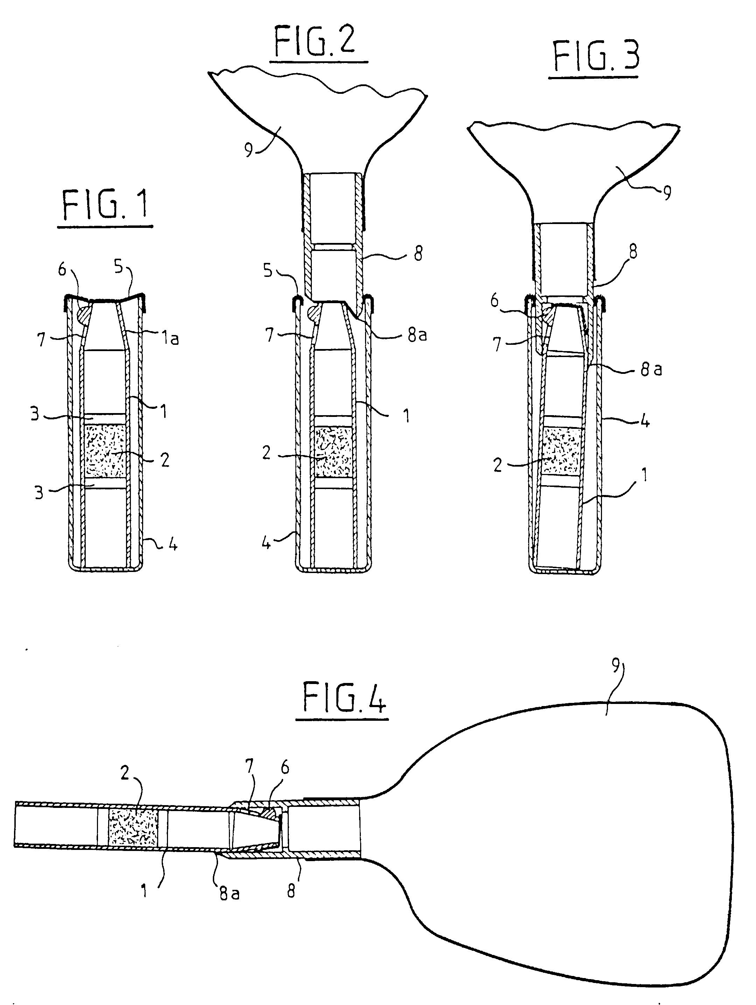

Pour utiliser le tube 1, on introduit dans le tube 4 un élément cylindrique 8 dont le diamètre extérieur est sensiblement égal au diamètre intérieur du tube 4 et dont le bord extérieur 8a est aminci et situé dans un plan oblique par rapport à l'axe de l'élément. Lors de cette introduction, le bord 8a découpe l'opercule 5 (Figure 2) et l'élément 8 s'emboîte de façon étanche dans le tube 1 (Figure 3); lors de cet emboîtage, la bossette 6 fait légèrement basculer le tube 1, ce qui dégage le trou 7; le fluide peut ainsi traverser l'élément 8 et la masse réactive du tube 1 lorsque ce tube a été extrait du tube protecteur 4, opération qui peut être faite sans contact manuel avec le tube 1.

Dans un mode de réalisation particulier, l'élément cylindrique 8 est fixé sur un appareil de mesure 9 délimitant le volume d'air à analyser; on peut ainsi déterminer le taux en alcool de l'air expiré par un être humain, donc son alcoolémie.

## Revendications

1. Dispositif pour déterminer le taux d'une substance donnée dans un fluide, par passage d'un volume déterminé de ce fluide dans un appareil de mesure (9) par l'intermédiaire d'un tube à essai (1) qui contient une matière ayant une réaction colorée avec cette substance et est, au stockage, disposé dans un tube de protection (4) qui a sensiblement même longueur que le tube d'essai et est obturé par un opercule imperméable (5),
caractérisé en ce que l'opercule (5) est fixé à la fois au tube à essai (1) et au tube de protection (2), de façon à immobiliser ces deux tubes l'un par rapport à l'autre et que la liaison de ce tube d'essai (1) avec l'appareil de mesure (9) s'effectue directement par traversée de l'opercule scellé (5).

2. Dispositif selon la revendication 1,
caractérisé en ce que l'extrémité (1a) du tube à essai (1) destinée à se trouver du côté de l'extrémité ouverte du tube de protection (4) est tronconique et présente une bossette extérieure (6) au-dessus d'un trou latéral (7).

3. Dispositif selon la revendication 2,
caractérisé en ce qu'il est associé à un élément d'extraction cylindrique (8) attenant à l'appareil de mesure (9) dont le diamètre intérieur est sensiblement égal au diamètre extérieur du tube d'essai (1) et dont le bord extérieur (8a) est aminci et coupant.

4. Dispositif selon les revendications 2 et 3,
caractérisé en ce que l'engagement de l'extrémité du tube à essai (1) dans l'élément d'extraction (8) par la bossette (6) libère un passage latéral pour l'écoulement du fluide à contrôler.

## Patentansprüche

1. Vorrichtung zur Bestimmung des Anteils einer in ein Fluid eingebrachten Substanz durch Einleiten eines vorgegebenen Volumens dieses Fluids in ein meßgerät (9) unter Zwischenschaltung eines Reagenzrohres (1), welches einen Stoff enthält, der zusammen mit dieser Substanz eine Farbreaktion zeigt, und welches zur Aufbewahrung in einem Schutzrohr (4) angeordnet ist, das im wesentlichen die gleiche Länge besitzt wie das Reagenzrohr und mit einem undurchlässigen Deckel (7) verschlossen ist, dadurch gekennzeichnet, daß der Deckel (5) gleichzeitig am Reagenzrohr (1) und am Schutzrohr (2) derart befestigt ist, daß diese beiden Rohre gegeneinander unbewegbar festgehalten sind und daß die Verbindung dieses Reagenzrohres (1) mit dem meßgerät (9) unmittelbar durch Durchdringen des versiegelten Deckels (5) bewirkt wird.

2. Vorrichtung nach Anspruch 1, dadurch gekennzeichnet, daß das Ende (1a) des Reagenzrohres (1), das dazu bestimmt ist, an der Seite des offenen Endes des Schutzrohres (4) zu liegen, kegelstumpfförmig ausgebildet ist und über einem seitlichen Loch (7) einen äußeren Vorsprung (6) aufweist.

3. Vorrichtung nach Anspruch 2, dedurch gekennzeichnet, daß ihr ein an das Meßgerät angrenzendes zylindrisches Extraktionselement (8) zugeordnet ist, dessen Innendurchmesser im wesentlichen gleich dem Außendurchmesser des Reagenzrohres (1) ist und dessen Außenrand (8a) angeschärft und schneidend ausgebildet ist.

4. Vorrichtung nach den Ansprüchen 2 und 3, dadurch gekennzeichnet, daß durch das Einstecken des Endes des Reagenzrohres (1) in das Extraktionselement (8) aufgrund des Vorsprungs (6) ein seitlicher Durchgang für das Abströmen des zu untersuchenden Fluids freigegeben wird.

## Claims

1. Device to determine the percentage of a given substance in a fluid via the passage of a specific volume of this fluid in a measuring device (9) by means of a test tube (1) containing a material having a coloured reaction with this substance and, when stored, is placed in a protective tube (4) having the same length as the test tube and being sealed off by an inner capsule (5), characterised in that the inner capsule (5) is fixed to both the test tube (1) and the protective tube (2) so as to render immobile these two tubes with respect to each other and in that the link of this test tube (1) with the measuring device (9) is effected directly via the crossing of the sealed inner capsule (5).

2. Device according to claim 1, characterised in that the end (1a) of the test tube (1) intended to be located on the side of the open end of the protective tube (4) is truncated and has an outer boss (6) located above a lateral hole (7).

3. Device according to claim 2, characterised in that it is associated with a cylindrical extraction element (8) contiguous to the measuring device (9) whose inner diameter is approximately equal to the outer diameter of the test tube (1) and whose outer edge (8a) is tapered and cutting.

4. Device according claims 2 and 3, characterised in that the engaging of the end of the test tube (1) inside the extraction element (1) via the boss (6) frees a lateral passage for flowing of the fluid to be inspected.
